Europäisches Patentamt

European Patent Office

Office européen des brevets

(11) Publication number: **0 404 458 A2**

# (12) EUROPEAN PATENT APPLICATION

(21) Application number: **90306561.3**

(22) Date of filing: **15.06.90**

(51) Int. Cl.5: **C12N 15/16, C12P 21/02, A61K 37/38**

(30) Priority: **19.06.89 AU 4799/89**

(43) Date of publication of application:
**27.12.90 Bulletin 90/52**

(84) Designated Contracting States:
**AT BE CH DE DK ES FR GB GR IT LI LU NL SE**

(71) Applicant: **Bunge (Australia) Proprietary Limited**
**6th Floor 616 St. Kilda Road**
**Melbourne Victoria 3004(AU)**

(72) Inventor: **Adams, Timothy Edward**
**48 Brassey Avenue**
**Rosanna, Victoria(AU)**
Inventor: **Brandon, Malcolm Roy**
**14 Castella Street**
**Ivanhoe, Victoria(AU)**

(74) Representative: **Harding, Richard Patrick et al**
**Arthur R. Davies & Co. 27 Imperial Square**
**Cheltenham GL50 1RQ(GB)**

(54) **Ovine follicle stimulating hormone.**

(57) A process for the preparation of a molecular clone includes providing a complementary DNA (cDNA) sequence, genomic DNA sequence or fragment thereof coding for the alpha and/or beta sub-unit of ovine follicle stimulating hormone, and a suitable cloning vector, and ligating the DNA sequence and cloning vector to form a molecular clone.

EP 0 404 458 A2

## Ovine Follicle Stimulating Hormone

The present invention relates to a synthetic follicle stimulating hormone (FSH), a process for the production of a synthetic FSH and a process for the construction of plasmid expression vectors for the expression and purification of high levels of ovine FSH in eukaryotic organisms.

FSH or follitropin, is a heterodimeric glycoprotein hormone secreted by the pituitary gland; and comprises a minor fraction of ovine polypeptide anterior pituitary proteins. FSH is made up of two parts, the so called alpha-subunit and the beta-subunit. The alpha-subunit is significantly more abundant than the beta-subunit since it is a common subunit in luteinizing hormone (LH) chorionic gonadotrophin (CG) and thyroid stimulating hormone (TSH). The alpha-subunit of these hormones is common within a species. The beta-subunit differs between these hormones and confers receptor specificity of each hormone, and biological activity.

FSH has a role in sexual function in both male and female animals. In males, it seems the most important function of FSH is in the maturation and nutrition of sperm, whilst in females FSH has a role in development of the ovum and ovulation. Exogenous FSH can be used for superovulation in embryo transfer programs, or inducing increased ovulation to give greater fecundity. However variation in the response of treated animals can be a major problem and may be attributed to batch variation and contaminants present in FSH preparations. The pituitary glycoprotein hormone LH is the major bioactive contaminant.

The major methods of ovine FSH extraction known in the prior art include

(i) the production of crude extracts of o-FSH from lamb pituitary glands by the use of ethanol and acid precipitation (Sherwood, et al (1970) J. Biol Chem. 245: 2329-2336, and

(ii) ammonium sulphate precipitation (Reichert, L.E. in Methods in Enzymology Vol. XXXVII ed. O'Malley B.W. and Hardman J.G. Academic Press Inc. N.Y. 1975).

Depending on the method used, unwanted proteins are precipitated out by the stepwise addition of cold ethanol or ammonium sulphate. A final addition of ethanol or ammonium sulphate precipitates the active component as well as some contaminants. These methods produce crude FSH extracts containing 0.5 - 1.0 units of o-FSH per mg of total protein.

Further purification steps using the crude pituitary extracts include gel filtration and ion-exchange chromatography (Sherwood, et al, 1970, Sairam 1979, Grimecti 1974). The purified products obtained range in potency from 110 to 188 units of o-FSH per mg of total protein. However, these procedures incur large expense, are time consuming and produce only small amounts from each pituitary gland. As a result large quantities of FSH are rarely available for veterinary use, especially FSH of a consistently high potency.

In the sheep, goat and cattle industries, administration of FSH may be utilized in a method of inducing or increasing ovulation in female animals. This is illustrated for example in copending Australian Patent Application No. 72219/87, the entire disclosure of which is incorporated herein by reference. However, whilst an economically viable role for FSH administration may be relevant with respect to the sheep, goat and cattle industries the supply of natural FSH falls well short of demand. Moreover, FSH exhibits some degree of species specificity. Sources of naturally occurring FSH other than ovine FSH have proved inferior in sheep, goat and cattle. However, developments in recombinant DNA technology and subsequent applications thereof allow for the manipulation of ovine FSH encoding DNA sequences allowing for the production of high levels of FSH in an eukaryotic organism such as a transgenic rabbit, or "in vitro" in eukaryotic cell systems.

Accordingly it is an object of the present invention to overcome, or at least alleviate, one or more of the difficulties related to the prior art. In particular, the process as described hereafter may produce relatively large quantities of recombinant polypeptides having good ovine FSH activity. The synthetic ovine FSH may be used in vivo as an effective substitute for the natural hormone.

Accordingly, in a first aspect of the present invention there is provided a process for the preparation of molecular clones which process includes;
providing complementary DNA (cDNA) sequences coding for the alpha and/or beta subunits of ovine FSH or fragments thereof; and
a suitable cloning vector;
ligating the DNA sequence and cloning vector to insert the DNA sequence into the vector to form a molecular clone.

The vector so formed may include a complete or partial copy of the ovine FSH coding region, encompassing the mature hormone, and extending past the 3′ terminating codon. The vector may include a copy of the alpha chain subunit and/or the beta chain subunit of the ovine FSH.

The DNA sequences coding for a polypeptide having ovine FSH activity may be derived from polyadenylated messenger RNA isolated from ovine lamb pituitary glands or from an ovine genomic DNA. The following description is illustrative only but is not intended to limit the scope in any way.

Accordingly, in a further aspect of the invention, there is provided a process for the preparation of DNA sequences coding for polypeptide having ovine follicle stimulating hormone activity, which method includes; providing a source of ovine lamb pituitary gland; isolating polyadenylated messenger RNA therefrom; and treating the polyadenylated messenger RNA to construct complementary DNA (cDNA) double stranded sequences.

The polyadenylated RNA isolation step from ovine pituitary gland may be achieved in any suitable manner, for example, by utilising a guanidine thiocyanate treatment and affinity chromatography.

The messenger RNA (mRNA) treatment step to form cDNA according to this aspect of the present invention may include treating the mRNA with reverse transcriptase in the presence of oligo dT primer to synthesize first strand cDNA; enzymatically removing the mRNA with RNase H, and subsequent synthesis of the second cDNA strand with DNA polymerase I; treatment of aforementioned double stranded cDNA with T4 DNA polymerase to create flush (blunt) ended molecules.

In a further aspect of the invention, there is provided a process for the preparation of ovine DNA sequences coding for a polypeptide having ovine follicle stimulating hormone activity, which method includes the preliminary steps of providing a source of ovine genomic DNA isolated from the sheep genome, and enzymatically cleaving the genomic DNA with a suitable enzyme.

The suitable vector according to the present invention may be selected from a variety of prokaryotic cloning vectors. The bacteriophage virus vector lambda gt10 and the like have been found to be suitable for cDNA cloning. Similarly the bacteriophage vector EMBL 3 has been found suitable for cloning genomic fragments.

The ligation step may take any suitable form. The cDNA cloning reaction step may be by means of ligation by the addition of synthetic DNA linkers. In this case linkers corresponding to the Eco RI restriction enzyme recognition sequence may be attached, utilizing T4 DNA ligase, to either end of the blunt ended cDNA or genomic DNA molecules.

Digestion of the aforementioned cDNA with the enzyme Eco RI will thus yield cDNA molecules with 5′ Eco RI cohesive overhanging termini. Internal EcoR1 sites are protected from digestion by methylation prior to linker addition.

Such molecules may then be cloned into the compatible Eco RI DNA site in, for example, the bacteriophage virus vector lambda gt10 by ligation; such DNA may then be packaged into virus particles "in vitro" and subsequently used to infect appropriate bacterial microorganisms to create a recombinant DNA library from which recombinant clones containing cDNA sequences or genomic DNA sequences encoding ovine FSH may be selected.

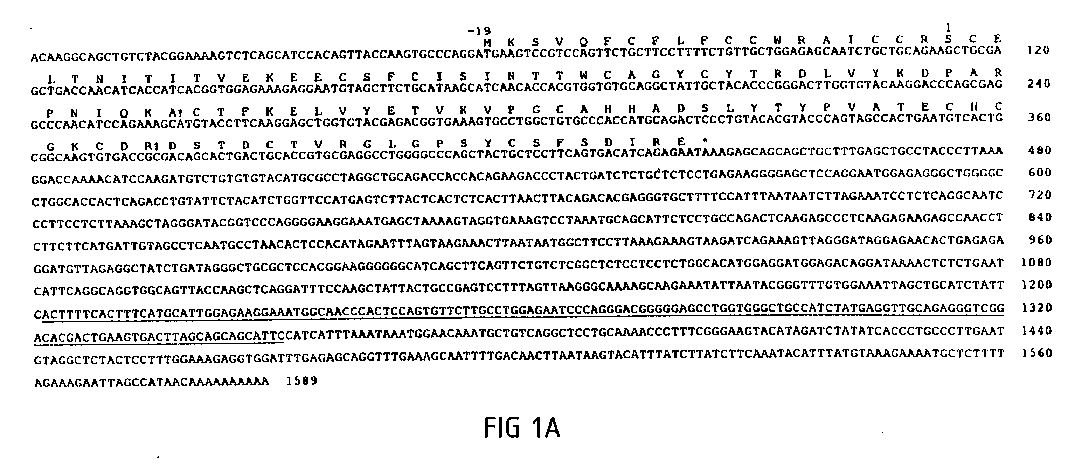

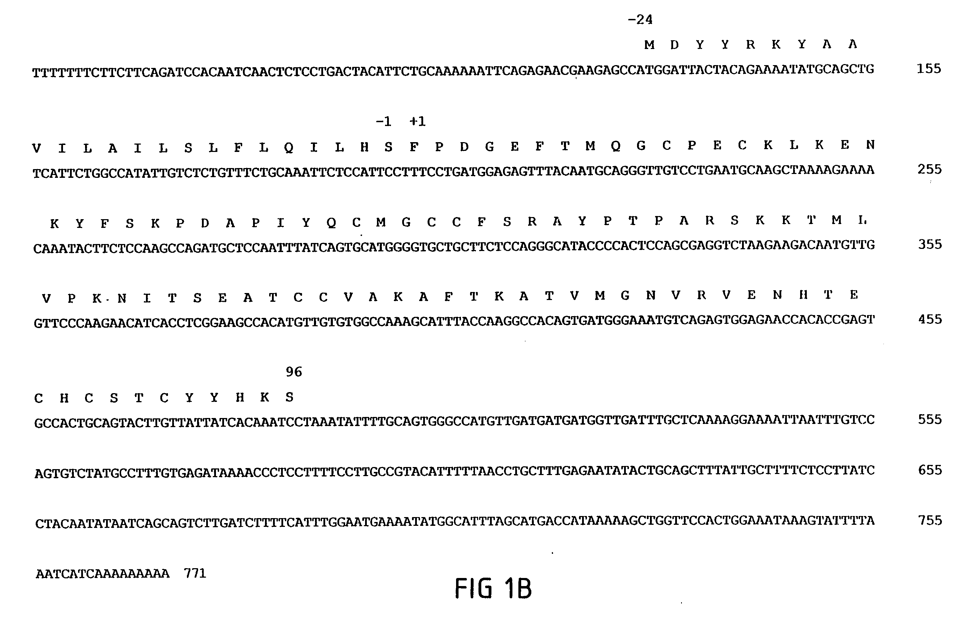

Accordingly, in a preferred aspect of the present invention the molecular clone may be a bacteriophage clone selected from the clones designated 3.2 and 6.14 as hereinafter described (see Figure 1A and 1B).

Samples of the bacteriophage clones are maintained in the collection maintained by Bunge (Australia) Pty. Ltd. North Melbourne, Victoria, Australia.

The alpha subunit clone, designated α3.2 or αFL, is 717 base pairs in length and includes 70 nucleotides of 5′ untranslated sequence, 72 nucleotides coding for the 24 amino acid signal peptide, 288 nucleotides coding for the 96 amino acid mature α polypeptide and 286 nucleotides of 3′ untranslated sequence.

The beta FSH subunit cDNA clone, designated β6.14 or βFL, is 1589 base pairs in length and includes the entire coding region for a 129 amino acid prehormone. The signal peptide is 19 amino acids in length. DNA sequence analysis of a second beta subunit cDNA clone has revealed an additional 3′ non-coding region extension of approximately 300 base pairs not seen in clone 6.14. When the predicted amino acid sequence is compared with that previously established by amino acid sequencing (Sairam, M.R., Seidah, N.G and Chretien, M. (1981)), two conservative substitutions and a single C-terminal amino acid exclusion were noted. The predicted amino acid sequence was confirmed by DNA sequencing of two independent cDNA clones. Comparison of the ovine nucleotide coding region sequence with the corresponding bovine sequence (Maurer, R.A. and Beck, A. (1986)) gives an overall homology of 95%. Comparison of the entire ovine beta-FSH cDNA clone 6.14, which includes a 149 bp 3′ non-coding region inclusion not seen in the bovine cDNA clone, gives an overall homology of 85%.

3

It has been shown that the presence of five ATTTA repeats distributed throughout the 3' non-coding region of the full length $\beta$ clone may be associated with destabliization of mRNA transcripts (Shaw et al., 1986).

Accordingly in a preferred aspect of the present invention, there is provided a cDNA sequence $\beta$SL cDNA which is a 573 bp EcoR1/Sac1 DNA fragment truncated at the 3' non-coding region, which no longer contains the five ATTTA repeats and has the DNA sequence illustrated in Figure 1A truncated at the Sac1 site.

There is provided in a further preferred aspect of the present invention a cDNA sequence designated $\alpha$SL cDNA comprising a truncated $\alpha$cDNA clone which has the 5' poly T cloning artifact, and some 3' non-coding region sequence removed from the $\alpha$FSH full length cDNA. This is a 607 bp Sau3A1/Sau3A1 DNA fragment which has the DNA sequence illustrated in Figure 1B truncated at the two Sau3A1 site.

It should be understood that while such bacteriophage vectors provide a useful vehicle for the stable maintainence of DNA sequences encoding polypeptide subunits having ovine FSH activity, such DNA sequences may be co-expressed in a eukaryotic expression system (e.g. mammalian cell lines and relevant plasmid vectors) to result in the biosynthesis of an ovine alpha/beta FSH heterodimer. Alternatively, subunits may be expressed individually using appropriate vectors, and assembled "in vitro" to yield a form of the hormone with biological activity.

Thus, one aspect of the present invention is a process for the preparation of a recombinant polypeptide having ovine FSH activity which process includes the steps of

providing
a a first recombinant plasmid including a plasmid expression vector including a complementary DNA (cDNA) sequence, genomic DNA sequence or fragments thereof coding for the alpha sub-unit of ovine FSH and capable of being transcribed and translated, and/or replicated in a unicellular organism,
a second recombinant plasmid including a plasmid expression vector including complementary DNA (cDNA) sequence, genomic DNA sequence or fragments thereof, coding for the beta sub-unit ovine FSH and capable of being transcribed and translated, and/or replicated in a unicellular organism,
a unicellular organism;
introducing said first and second recombinant plasmids into said unicellular organism by a method selected from transformation or transfection;
culturing the resulting organism;
expressing the recombinant polypeptide encoded by said DNA sequences; and optionally
isolating said polypeptide from the culture.

In a further aspect of the present invention there is provided a plasmid expression vector including a cDNA sequence, genomic DNA sequence or fragments thereof coding for the alpha-subunit ovine FSH and capable of being replicated, transcribed and translated in a unicellular organism.

In a still further preferred aspect of the present invention there is provided a plasmid expression vector including a cDNA sequence, genomic DNA sequence or fragments thereof coding for the beta-subunit ovine FSH and capable of being transcribed and translated, and/or replicated in a unicellular organism.

Preferably the plasmid expression vector includes
a first cDNA sequence, genomic DNA sequence or fragments thereof coding for the alpha-subunit of ovine FSH, and
a second cDNA sequence, genomic DNA sequence or fragments thereof coding for the beta-subunit of ovine FSH; and
capable of being replicated, transcribed and translated in a prokaryotic or eukaryotic organism.

Thus a plasmid expression vector is provided for expression of the relevant cDNA or genomic DNA sequences coding for a polypeptide having ovine FSH activity, such DNA sequences may also be co-expressed in a eukaryotic expression system such as a transgenic animal to result in the biosynthesis of an ovine alpha-beta FSH heterodimer. The vector may contain a promoter sequence, preferably a metallothionein promoter, more preferably a human metallothionein promoter and most preferably an 841 base pair human metallothionein promoter $II_A$ (h $MTII_A$).

The promoter sequence which, may be the $hMTII_A$ promoter HindIII/BamH1 841 bp fragment may be directionally cloned into HindIII/BamH1 digested pSP72 (Promega Corporation). Accordingly there is provided a plasmid expression system designated F1 which is an pSP72 vector containing the human metallothionein promoter.

An enhancer element may be included upstream from the $hMTII_A$ in the plasmid expression system FI. The enhancer element may be an SV40 enhancer element, preferably the 840 bp Hind III/Eco RV enhancer region, and may be directionally cloned into FI by primarily digesting with HindIII/Pvu II, dephosphorylating and ligating by known methods. Accordingly there is provided a vector designated FII which is pSP72 containing the hMTII$_A$ promoter and an upstream SV40 enhancer region.

The SV40 enhancer fragment contains the SV40 origin of replication thereby making the vector suitable for transient COS cell expression studies (Gluzman, 1981) as well as constitutive expression in other cell lines such as Chinese hamster ovary cells (CHO) (Friedman, et al. 1989).

An SV40 derived BamH1 fragment containing the small t intron and polyadenylation signal may also be ligated into a unique BglII site of the FII vector. Accordingly, there is provided in the present invention a plasmid expression vector designated FIII which results from the ligation of a SV40 derived BamHI fragment containing the small t intron and polyadenylation signal, SV40 enhancer fragment and a hMTII promoter sequence into pSP72.

Thus, it is provided in the present invention the following clones FIIIαFL, FIIIβFL, FIIIαSL, FIIIβSL containing αFL, βFL, αSL and βSL ligated into FIII respectively.

DNA sequence analysis of the βFSH cDNA has shown that the translation initiation codon flanking regions contain suboptimal sequence arrangements for translation initiation (Kozak, 1986). In vitro mutagenesis experiments have established the optimized translation initiation DNA sequences as ACCATGG and GCCATGG (Kozak, 1986). Replacement of the existing AGGATGA initiation codon region may increase the translatability of the β chain mRNA. Insertion of such translation initiation sequences into truncated βFSH may be attained by using oligonucleotide mediated in vitro mutagenesis or any other suitable technique to achieve the desired result.

Thus, in a further preferred aspect of the present invention there is provided two clones FIIIβSL(M2) and FIIIβSL(M12) designed to increase translatability of the β chain mRNA. FIIIβSL(M2) contains the sequence ACCATGG while FIIIβSL(M12) contains GCCATGG incorporated into the truncated βFSH subunit cDNA clone FIIIβSL.

Thus hybrid DNA sequences may be constructed. The hybrid DNA sequences may enable other DNA sequences to be linked to a DNA sequence which codes for a desired protein. The hybrid DNA sequences so constructed may enhance translatability of mRNA wherein the DNA sequence contains initiation sequences obtained from a non-related gene. By adding such a cDNA sequence containing a translation initiation sequence, a 5′ non-coding region and a signal peptide coding region upstream to a gene encoding a desired protein, translatability may be enhanced.

Transcription initiation sequences may also be incorporated in such sequences if the DNA is obtained from a genomic DNA library.

Thus, in a further aspect of the present invention there is provided a plasmid including

a plasmid expression vector;

a first DNA sequence encoding a desired protein; and

a second non-related DNA sequence including a translation initiation and optionally transcription initiation region;

a 5′ non-coding region; and

a signal peptide coding region; the second DNA sequence being positioned upstream of said first DNA sequence.

In a further preferred aspect the second DNA sequence may be derived from a DNA sequence encoding a growth hormone and more preferably an ovine growth hormone.

The first DNAs equence may encode an ovine follicle stimulating hormone; preferably an ovine follicle stimulating hormone.

Hence, in order to increase translatability of the βchain mRNA the entire 5′ non-coding and signal peptide coding region of βFSH cDNA may be replaced with the equivalent region of the ovine growth hormone cDNA.

An ovine growth hormone βFSH cDNA hybrid may be engineered into plasmid FIII described above. The resultant clone contains a 120 bp fragment of the ovine growth hormone cDNA containing both the 5′ non-coding and signal peptide regions ligated to a truncated βFSH which is a truncated βSL fragment restricted at

(i) the PvuII site upstream from the Sac1 site thereby removing 120 bp from the 3′ end, and

(ii) the 5′ Pst1 site thereby removing 110 bp from the 5′ end.

Accordingly, there is provided in a preferred aspect of the present invention the clone FIIIβSL(T4).

It should also be understood that expression of biologically active recombinant o-FSH is not restricted to the genetic manipulation of relevant cDNA clones although this is the preferred form of this aspect of the invention described. The expression of FSH from cloned, genomic DNA fragments isolated from the sheep genome represents an alternate approach that is not excluded by the following technology described herein. It should be understood that expression of the relevant cDNA or fragments of genomic clones may be undertaken both in the preferred eukaryotic and alternate prokaryotic expression systems.

Accordingly, in accordance with a further aspect of the present invention there is provided a method of inducing or increasing ovulation in female animals which method includes administering to the animal an effective amount of a ovine synthetic FSH, or derivative thereof, as hereinbefore described.

The animals to be treated may be selected from sheep, goats, cattle, deer, dogs, cats, pigs and the like.

The method of treating the female animal may be of any suitable type. The treatment may be oral, by injection, by implant or the like. Accordingly, the FSH may be provided in a unit dosage form. An oral form, an injectable form or an implant form may be used. The FSH may be in a lyophilised form.

It has been surprisingly found that ovine FSH provides an improved yield of ova relative to other treatments including treatment with p-FSH. Moreover, it has been found that embryos produced from ova induced by ovine FSH are of improved quality relative to prior art treatments and damage to the ovaries of the treated animals is surprisingly reduced. The embryos so produced are characterised by an increased viability.

The amount of o-FSH hormone to be used will vary with the purity and origin of the ovine FSH (natural or synthetic), the species of animal to be treated and the reproductive function to be achieved. Synthetic ovine FSH has a higher purity relative to o-FSH isolated from natural sources and thus may be administered at substantially reduced weight dose rates. Accordingly, in a preferred form the present invention provides a method of increasing the number of ova produced during ovulation in female animals, which method includes administering to the animal an effective amount of a synthetic ovine FSH for a period sufficient to induce increased ova production.

The individual dose rates may vary from approximately 8 units to approximately 60 units. The biological measurement of a "unit" is described by Stedman & Pohley (Endocr. 53, 604-616, 1953) and Maghuir-Rogister et al (Eur. J. Biochemistry, 86, 121-131, 1978) and represents a standard measure of biological activity. The treatment may comprise a single dose. The treatment may continue for approximately 1 to 10 days. The selection of dose rates is dependent on the size, age and species of animal to be treated. It has been found suitable to reduce the dosage rate over the treatment period. An increased dosage regimen may be utilized for cattle.

Accordingly in a preferred form there is provided a method of increasing the number of ova produced during ovulation in female animals which method includes administering to the animal a synthetic ovine FSH, as hereinbefore described, wherein when the animal is a bovine animal the hormone is administered regularly at least 3 times a day in substantially equal doses to a total amount of from approximately 20 to 60 u, dependant upon the size of the animal; and

when the animal is an ovine or caprine animal the hormone is administered in an amount of from approximately 20 to 40 U in a single dose.

In a preferred form of this aspect of the present invention there is provided a method of increasing ovulation to provide twinning in female cattle.

The treatment may comprise a single dose or the treatment may continue over a period of approximately 2 to 4 days. The dose rate may be given in reducing amounts.

In a preferred form the dosage regimen includes administration of synthetic ovine FSH in 1 to 5 doses over a period of one day. It has been surprisingly found that twinning may be induced in cattle on a relatively consistent basis utilising natural FSH in cattle. It will be understood that twinning is ideal in cattle production. The production of a single calf is an inefficient use of resources, whereas the production of 3 or more ova and thus embryos may often result in deformities and/or spontaneous abortion in cattle. Preferably, the cattle treated are sexually mature. Preferably wherein the animal is bovine and ovine FSH is administered in three substantially equal amounts totalling approximately 0.06 to 0.24 U/kg live weight in a single day, more preferably the total dose rate is approximately 0.09 to 0.15 U/kg live weight in a single day.

Alternatively wherein the animal is a sheep or goat synthetic ovine FSH is administered in an amount of approximately 20 to 30 U in a single dose.

In an alternative form of this aspect of the present invention there is provided a method of inducing ovulation in pre-pubescent female animals which method includes administering to an animal an effective amount of a synthetic o-FSH, or derivative thereof, for a period sufficient to induce ovulation.

The pre-pubescent animals may be treated with a single dose. The pre-pubescent animals may be treated at reducing dosage rates over a period of approximately 1 to 5 days.

In a preferred form, the method of inducing or increasing ovulation to produce twinning or otherwise, may include the preliminary step of treating the animal with an effective amount of a synchronising agent. The synchronising agent may be selected from any of the agents known per se. The agent may be a progestagen, prostaglandin, prostaglandin analogue or the like. Synchronising agents sold under the trade designation Chronogest (available Intervet Australia) and "Repromap" (available from Upjohn Pty. Ltd.) intravaginal sponges have been found to be suitable in sheep and goats. The synchronising agent sold under the trade designation Estrumate (available from ICI Australia Ltd.) has been found to be suitable for cattle.

The preliminary treatment with the synchronising agent or the like may be undertaken at a preselected interval of time prior to the initiation of treatment with the o-FSH. The treatment with synchronising agent may be undertaken approximately 8 to 15 days prior to FSH treatment.

It has been found that the synchronising treatment in combination with the ovine FSH treatment was effective in inducing ovulation in pre-pubescent female cattle of approximately 10 months of age.

Preferably approximately 25 mg to approximately 100 mg of the synchronising agent is administered to a pre-pubescent animal approximately 8 to 15 days prior to initiation of ovine FSH treatment. The treatment with synchronising agent may be repeated at a suitable interval if necessary.

In a more preferred form, the method of inducing or increasing ovulation to produce twinning or otherwise may include the further step of treating the animal with an effective amount of a luteinizing agent during or after the synthetic o-FSH treatment. The luteinizing agent may be a prostaglandin or prostaglandin analogue. The luteinizing agent may be the same as, or different to the synchronising agent previously described.

Preferably the luteinizing agent is a prostaglandin or prostaglandin analogue and is administered in an amount of approximal 250 mg to 1000 mg approximately 1 to 3 days after the initiation of ovine FSH treatment.

In a further preferred form the method of inducing or increasing ovulation may include the further step of administering to the animal an effective amount of gonadotrophin or derivative thereof.

It has been found that some animals in some trials, of the order of 20%, do not respond to the o-FSH treatment done. It has now been found that an additional treatment with a relatively small amount of gonadotrophin or derivative thereof results in a substantial reduction of the number of animals who are not responding.

A pregnant mare serum gonadotrophin (PMSG) may be used. A pregnant mare serum gonadotrophin of the type described in Australian patent application 80031/87 filed 14th October, 1987 has been found to be suitable (the entire disclosure of which is incorporated herein by reference). More preferably the monoclonal antibody derived therefrom disclosed therein may also be used. PMSG is also referred to as equine chorionic gonadotrophin (eCG).

The gonadotrophin or derivative thereof may be administered in amounts of from approximately 100 I.U. to 1000 I.U. and preferably 500 I.U.

The gonadotrophin treatment may be administered at any time during or before the ovine FSH treatment. Preferably the gonadotrophin treatment may be undertaken on the first day of the ovine FSH treatment.

In a further aspect of the present invention there is provided a veterinary composition including an effective amount of a synthetic o-FSH, or derivative thereof, as hereinbefore described; and a veterinary composition may further include a veterinarily acceptable carrier or excipient therefor.

The ovine FSH or derivative may be provided in a lyophilised form. The veterinary composition may further include a veterinarily acceptable carrier or excipient. The carrier or excipient may be a solvent for the follicle stimulating hormone or derivative. An aqueous solvent may be used. A physiologically acceptable saline buffered solvent may be used. The veterinary composition may be provided in an injectable form.

In a preferred form of this aspect of the present invention there is provided the veterinary composition further includes an effective amount of a gonadotrophin or gonadotrophin analogue.

In a preferred form the present invention provides a kit of parts including a supply of synthetic ovine FSH in a lyophilised form in a suitable container.

The kit of parts may further include a supply of a synchronising agent in a suitable container. The ovine FSH, gonadotrophin, and the synchronising agent where present, may be provided in an injectable form.

The present invention will now be more fully described with reference to the following examples. It should be understood, however, that the description following is illustrative only and should not be taken in any way as a restriction on the generality of the invention described above.

In the figures:

FIGURE 1A is a nucleotide sequence and predicted amino acid sequence of the ovine FSH beta-subunit precursor hormone cDNA clone 6.14. The position of the C-terminal amino acid exclusion is between the second last and third last amino acids. The termination codon is indicated by the asterisk. The 149 bp inclusion is underlined. The amino acids differing from the published peptide sequence (Sairam, M.R., Seidah, N.G. and Chretien, M. (1981)) are indicated by +.

FIGURE 1B shows a nucleotide sequence and predicted amino acid sequence of the ovine FSH alpha-subunit precursor hormone cDNA clone 3.2.

FIGURE 2 is an SDS-PAGE analysis of immunoprecipitated $^{35}$S (Cysteine) metabolically labelled rFSH (recombinant FSH) expressed in COS cells.

## EXAMPLE 1

### Preparation of the ovine FSH alpha and beta subunit cDNA clones

FSH comprises a minor fraction of ovine polypeptide anterior pituitary proteins; the alpha chain subunit is significantly more abundant than the beta chain subunit, as it is a common subunit in luteinizing hormone (LH) and thyroid stimulating hormone (TSH). The strategy for obtaining cDNA clones for both alpha and beta subunits was to use the entire mRNA population as a template for cDNA synthesis, and to select from the population, using specific oligonucleotides, clones encoding the alpha and beta chain FSH subunits.

In summary this involved:

(1) Isolation of total cellular RNA from ovine pituitary using a guanidine thiocyanate based method and the further purification of poly A$^+$ messenger RNA on oligo (dT) cellulose.

(2) Synthesis of cDNA using a two-step protocol utilising
(i) reverse transcriptase, and
(ii) RNase H/DNA polymerase I.

(3) Cloning into EcoR1-restricted lambda gt10 using synthetic linkers, followed by "in vitro" packaging of the DNA and infection of E.coli NM514.

### Isolation of Pituitary RNA

Frozen (-70°C) ovine lamb pituitary glands were homogenised using a Polytron homogeniser in 5M guanidine thiocyanate, 1% sarkosyl, 20 mM EDTA, 1% (v/v) 2-mercaptoethanol, 50 mM Tris-HCl, pH 7.8 (1 gram) pituitary/10 mls guanidine thiocyanate solution). Homogenates (10 ml) were layered over 4 ml of 5.7M CsCl containing 0.1 M EDTA, pH 7.0, in a Beckman SW28.1 centrifuge tube, and centrifuged at 22,000 rpm for 20 hr at 20°C. The supernatant was carefully aspirated with a Pasteur pipette, and the RNA pellet dissolved in 0.5 ml of RNase-free, sterile H$_2$O. Samples were extracted twice with phenol-chloroform, twice with chloroform and RNA precipitated at -20°C by the addition of 0.1 volume of 3M sodium acetate, pH 5.2 and 2.5 volumes of ethanol (adapted from Chirgwin et al., Biochemistry 18: 5294, 1979).

### Selection of Poly(A) + RNA

Messenger RNA, or more precisely polyadenylated RNA, can be isolated from a mixture of RNA species by chromatography on oligo (dt) (dT-cellulose). Oligo-(dT) (dT-cellulose), Type 7, was purchased from Pharmacia. The resin was equilibrated in sterile loading buffer (20 mM Tris-HCl (pH 7.6), 0.5M NaCl, 1mM EDTA, 0.1% SDS) and a 1.0 ml column poured. The column was washed three times with each of a) sterile H$_2$O b) 0.1M NaOH/5mM EDTA and c) sterile H$_2$O (or until the column effluent pH was less than 8). Five volumes of sterile loading buffer were then passed through the column. The RNA was prepared by suspension in sterile H$_2$O and heat-treated at 65°C for 5 minutes. An equal volume of 2 x loading buffer was added to the RNA sample, and then allowed to cool to room temperature before application to the column. Column flow-through was collected, heated to 65°C for 5 minutes, cooled and reapplied to the column. The column was then washed with 5-10 column volumes of loading buffer, followed by 4 column volumes of 20mM Tris-HCl (pH 7.6) 0.1M NaCl, 1mM EDTA, 0.1% SDS. The polyadenylated RNA was eluted with 2-3 column-volumes of sterile 10mM Tris-HCl (pH 7.5), 1mM EDTA, 0.05% SDS and precipitated with 3M sodium acetate and ethanol. The pellet was resuspended in sterile H$_2$O and stored at -70°C. Elution of polyadenylated RNA was followed by reading the optical density at 260 nm.

## Synthesis of Complementary DNA (cDNA)

Poly A⁺ messenger RNA was converted into double-stranded cDNA using the modifications described by Gubler, U. Nucleic Acid Research 16:2726 (1988) and inserted into the bacteriophage vector lambda gt10 according to the protocols outlined in "Basic Methods in Molecular Biology" -L.G. Davis et. al. Briefly this involved oligo dT-primed synthesis of the first cDNA strand from the mRNA template using reverse transcriptase (RNA-dependent DNA polymerase), removal of the RNA template by RNase H, synthesis of the second strand with E.coli DNA polymerase I and the subsequent blunt-ending of the cDNA using T4 DNA polymerase.

## Synthesis of First and Second Strands

1-2 ug of polyA⁺ RNA was heated to 65°C for three minutes, then cooled on ice. First strand cDNA was synthesized in a final volume of 20 ul of 50mM Tris-HCl, pH 8.3, 10mM $MgCl_2$, 10 mM DTT, 100 mM NaCl, 4 mM Na Pyrophosphate, 1 mM dNTP's (dATP, dGTP, dCTP, dTTP), 100 ug/ml oligo $dT_{12-18}$, 1-2 ug polyA⁺RNA and 20 U/ug RNA AMV reverse transcriptase at 42°C for 40 minutes. The reaction was cooled on ice. 50 ul of 2 x second strand buffer (40 mM Tris-HCl, pH 7.5, 10 mM $MgCl_2$, 20 mM $(NH_4)_2SO_4$, 200 mM KCl, 100 ug/ml BSA) containing 4.6 u of RNase H and 115 u DNA polymerase I and 10 ul of $H_2O$ were then added to give a final volume of 100 ul. The reaction was incubated at 12°C for 60 minutes at 22°C. The reaction was terminated by heating at 70°C for 10 minutes. After cooling on ice, T4 DNA polymerase was added (2 u) and the contents incubated at 37°C for 10 minutes. EDTA was added to a final concentration of 20 mM; the double stranded cDNA was then phenol-chloroform extracted twice, and precipitated twice with sodium acetate and ethanol. Quantitative analysis of the efficiency of first and second strand cDNA synthesis was monitored by the incorporation of $^{32}P$-dATP into acid-precipitable material within each of the respective reactions (i.e. measuring the conversion of free nucleotide into acid-precipitable DNA). Qualitative analysis of first and second strand reaction products was performed by analysing aliquots of the respective reactions on denaturing alkaline agarose gels.

## Cloning Double Stranded cDNA

The pituitary cDNA is ligated to blunt ended EcoR1 synthetic DNA linkers, subsequently cut with the enzyme EcoR1 to create EcoR1 cohesive end termini, and finally purified by column chromatography prior to being ligated to gt10 DNA.

The reactions are performed as follows:

(i) 200 ng of ethanol-precipitated cDNA is pelleted by centrifugation at 13,000 g in an Eppendorf centrifuge. The cDNA pellet is washed with cold 70% ethanol, air dried, and resuspended in 14 ul of sterile $H_2O$. Synthetic EcoR1 linkers (8-mer phosphorylated, purchased from New England Biolabs) are resuspended in 10mM Tris-HCl, pH 8.0 1 mM EDTA at a concentration of 500 ug/ml and 2 ul added to the cDNA. 2 ul of 10x ligase buffer (300 mM Tris-HCl, pH 7.4, 100 mM $MgCl_2$, 100 mM DTT, 10mM ATP) and 2 ul of T4 DNA ligase are added and the reaction allowed to proceed overnight at 15°C. The ligase is inactivated by heating at 70°C for 10 minutes; then 10 ul EcoR1 enzyme buffer (10 x; 700 mM Tris-HCl, pH 7.4, 500 mM NaCl, 50 mM $MgCl_2$, $H_2O$), water and 100 u of EcoR1 are added to a final volume of 100 ul. The reaction is allowed to proceed at 37°C for 4 hours. The reaction mixture is then heat inactivated by incubating at 70°C for 10 minutes. cDNA is separated from free linkers by size exclusion chromatography on a 2 ml column containing Sepharose CL-4B (Pharmacia) equilibrated in STE (100 mM NaCl, 10 mM Tris-HCl, 1 mM EDTA, pH 7.5). Fractions containing cDNA are collected, pooled and salt-ethanol precipitated overnight at -20°C. The cDNA is pelleted by centrifugation, washed in cold 70% ethanol, air dried and resuspended in distilled $H_2O$.

(ii) 20 ng of cDNA is ligated to 1 ug of EcoR1 digested, dephosphorylated gt10 arms (Amersham) in a final volume of 10 ul of ligation buffer (30 mM Tris-HCl, pH 7.4, 10 mM DTT, 10 mM $MgCl_2$, 1 mM ATP) containing 2 u of T4 DNA ligase. This is then packaged into bacteriophage viral particles using "in vitro" packaging reactions, obtained from Promega Biotec Pty. Ltd., according to the manufacturers instructions.

A single bacterial colony of the E.coli strain NM514 is inoculated into 10ml of L broth (1% yeast extract, 1% tryptone, 0.5% NaCl, pH 7.5) supplemented with o.4% maltose, and grown overnight at 37°C. 500 ul of this culture is then added to 50 ml of L broth plus 0.5% maltose, and cultured at 37°C until an $O.D._{600}$ of 0.5 is reached. The culture is then centrifuged at 2,000 g for 10 minutes, the supernatant discarded, and the bacterial pellet resuspended in 12.5 ml of cold 10mM $MgSO_4$. Aliquots of packaged, cDNA-containing bacteriophage particles are absorbed to 100 ul suspensions of NM514 cells by incubation at room temperature for 20 minutes. Three ml of molten agarose (0.7% in L broth; equilibrated to 47°C) is added to each suspension, mixed and poured onto 92 mm petri dishes containing 1.5% agar in L broth. Plates are then incubated for 8 - 10 hours after which zones (plaques) of recombinant lambda gt10 bacteriophages can be seen growing against a background bacterial lawn of NM514 cells.

Recombinant bacteriophages containing cDNA copies encoding for both alpha and beta FSH subunits were identified by hybridisation to $^{32}$P-radiolabelled oligo nucleotides. Briefly, nitrocellulose fitters were overlaid onto bacterial lawns containing recombinant bacteriophages; the filters were removed and adhering phage particles lysed under alkaline conditions to release DNA, which was subsequently fixed to the filter, after neutralisation, by uv irradiation (cross-linking) for 2-3 minutes. Filters were incubated for 16 hours at 40°C with an aqueous solution containing $^{32}$P-radiolabelled oligonucleotides. They were then washed in 2 x SSC, 0.1% SDS, dried and exposed to autoradiographic film. Positive colonies were identified, plaque purified and DNA isolated from bacteriophage suspensions. Phage DNA were digested with EcoR1, and cDNA inserts isolated by electrophoresis on low-melting temperature agarose gels, followed by phenol-extraction and ethanol/salt precipitation. Purified inserts were subcloned into the ampicillin-resistant plasmid vector pUC18.

## DNA Sequence of alpha and beta FSH cDNA Clones

Two cDNA clones 3.2 and 6.14 were chosen for further analysis. Sequence analysis was performed using the Sanger dideoxy sequencing method, as adapted for sequencing double stranded DNA inserts cloned into the pUC series of plasmid vectors (USB Comments 14 (4): 18-20, 1988).

Results are presented in Figure 1. cDNA clone 6.14 encodes a full length copy of mRNA encoding ovine FSH-beta subunit polypeptide, and includes 5′ and 3′ non-coding sequences. Clones coding for the beta-subunit of ovine FSH were selected from an ovine lamb pituitary lambda-gt10 cDNA library using two synthetic oligonucleotide probes. Both probes were synthesised on the basis of published amino acid sequence for the FSH beta-subunit (Sairam, M.R., Seidah, N.G. and Chretien, M. (1981)). The complete DNA sequence for the ovine FSH-beta clone 6.14, determined by dideoxy sequence analysis (Sanger, F., Nicklen, S. and Coulson, A.R. (1977)) and exonuclease III digestion (Henikoff, S. (1984)), is presented in Figure 1A.

The clone is 1589 base pairs in length and includes the entire coding region for a 129 amino acid FSH beta-subunit prehormone. The signal peptide is 19 amino acids in length. When the predicted amino acid sequence is compared with that previously established by amino acid sequencing (Sairam, M.R., Seidah, N.G. and Chretien, M. (1981)), two conservative substitutions and a single C-terminal amino acid exculsion were noted. The predicted amino acid sequence was confirmed by DNA sequencing of two independent cDNA clones. Comparison of the ovine nucleotide coding region sequence with the corresponding bovine sequence (Maurer, R.A, and Beck, A. (1986)) gives an overall homology of 95%. Comparison of the entire ovine beta-FSH cDNA clone 6.14, which includes a 149 bp 3′ non-coding region inclusion not seen in the bovine clone, gives an overall homology of 85%.

Similarly the cDNA clone 3.2 (Figure 1B) encodes the full polypeptide coding region of the common alpha-subunit of FSH, including 5′ and 3′ non coding regions.

## EXAMPLE 2

Ovine FSH has alpha and beta subunit peptides of 96 and 110 amino acids respectively. Ovine cDNA clones encoding the alpha and beta subunit peptides have been isolated from an ovine lamb pituitary cDNA library (Bello et al. 1989, Mountford et al. 1989) using oligonucleotide probes synthesised on the basis of the published amino acid sequences (Sairam 1983, Sairam et al, 1981).

## Construction of the FIII Eukaryotic Expression Vector

Expression vector constructs were designed as follows. The final vector designated FIII contains the 841 base pair human metallothionein $II_A$($hMTII_A$) promoter and an upstream SV40 enhancer element.

Briefly, the hMTII$_A$ promoter HindIII/BamH1 841 bp fragment was directionally cloned into HindIII/BamH1 digested pSP72 (Promega Corporation) to produce the FI vector. FI was digested with HindIII/PvuII and dephosphorylated, and the 840 bp HindIII/EcoRV SV40 enhancer region was then cloned into this site to produce the vector designated FII. An SV40 derived BamH1 fragment containing the small t intron and polyadenylation signal was then ligated into the unique BglII of the FII vector to produce the vector designated FIII. The SV40 enhancer fragment contains the SV40 origin of replication thereby making the vector suitable for transient COS cell expression studies (Gluzman, 1981) as well as constitutive expression in other cell lines such as Chinese hamster ovary cells (CHO) (Friedman et al 1989).

Construction of FIII Alpha and Beta FSH Subunit Expression Vector Constructs

Full length and truncated or short length cDNA inserts for alpha and beta FSH subunit coding regions were cloned into FIII.

The truncated $\beta$FSH clone no longer contains the five ATTTA repeats found distributed throughout the 3' non-coding region of the full length clone. This is significant in that these repeats have previously been associated with destabilization of mRNA transcripts (Shaw et al. 1986). This vector was designated FIII$\beta$SL. Truncation of the alpha cDNA clone removed a 5' polyT cloning artifact and hence 3' non-coding region sequence from the alpha FSH cDNA clone. This vector was designated FIII$\alpha$SL.

Two additional truncated $\beta$FSH subunit mutants were constructed in an attempt to increase the translatability of the $\beta$ chain mRNA. Sequence analysis of the $\beta$FSH cDNA showed that the transcription initiation codon flanking regions contained what have been reported to be suboptional sequence arrangements for translation initiation (Kozak, 1986). In vitro mutagenesis experiments (Kozak, 1986) established the optimised translation initiation DNA sequences as ACCATGG and GCCATGG. These sequences were incorporated into the truncated $\beta$FSH subunit cDNA clones using oligonucleotide mediated in vitro mutagenesis as previously described (Amersham publication code RPN 1523).

The vector containing the sequence ACCATGG was designated FIII$\beta$SL(M2) and the other containing GCCATGG was designated FIII$\beta$SL(M12).

An ovine growth hormone/$\beta$FSH hybrid DNA was constructed in an attempt to increase translatability of the $\beta$ chain mRNA. The 5' non-coding region and signal peptide of the $\beta$ subunit was replaced by the equivalent region from the ovine growth hormone and inserted into FIII for expression under direction of the metallothionein promoter. The details of construction are as follows.

Isolation of the ovine growth hormone leader sequence

An 860 bp ovine growth hormone fragment was isolated from pUC18/oGH by digestion with PvuII. The fragment contains the 5' non-coding region and the signal peptides of oGH (ovine growth hormone) along with a further 560 bp of the oGH. The fragment was dephosphorylated prior to agarose gel electrophoresis isolation and purification by known methods.

Following digestion with AhaII the fragment was filled in with Klenow and then further digested with EcoR1 to generate a 120 bp fragment containing primarily the 5' non-coding and signal peptide coding regions. The oGH fragment was then available for ligation into the $\beta$FSH containing vector. The remaining 180 bp fragment containing pUC18 sequences had been previously dephosphorylated to prevent ligation into the $\beta$-FSH containing vector.

Construction of the $\beta$FSH mature peptide coding sequence and vector

pUC18 was digested with Pst1, blunt ended with T4 (DNA polymerase) and dephosphorylated. A truncated $\beta$FSH SL PvuII fragment (453 bp) which lacks 120 bp of 3' non-coding region and 7bp of 5' non-coding region sequence, was ligated into the blunt ended Pst1 site of pUC18. The $\beta$FSH fragment containing one Pst1 site was digested with the same enzyme (Pst1) and blunt ended with T4 DNA polymerase, heat inactivated and dephosphorylated. The open ended pUC18 vector was further digested with EcoR1 at a site further upstream from the Pst1 site thereby liberating pUC18 sequences and creating an EcoR1 site for shot gun ligation of the oGH fragment isolated from pUC18/oGH as previously described.

The resulting vector contains the 5' non-coding sequences and signal peptides of pGH ligated to a truncated $\beta$SL in pUC18. The resulting sequence acquires an additional glycine at the interface of the pGH leader sequence and the βSL mature peptide coding sequence.

## Construction of the FIII βSL (T4)

The pUC18 containing oGH (leader)/βSL hybrid DNA as previously described was digested with EcoR1/HindIII and inserted into pSP72. From this vector an EcoR1/PvuIII fragment is obtained following digestion, then insertion of this fragment into an EcoR1/EcoRV site in FIII results in the final vector, FIII/βSL (T4).

## COS Cell Transient Expression

COS cells were transfected with either
(i) FIII vector alone.
(ii) FIII αSL
(iii) FIII βSL
(iv) FIII αSL + FIII βSL
(v) FIII αSL + FIII βSL(M2)
or
(vi) FIII αSL + FIII βSL(T4). via Lipofectin mediated DNA transfection (Felgner et al., 1987) and expanded for 48 hours prior to overnight metabolic labelling with $^{35}$S cysteine.

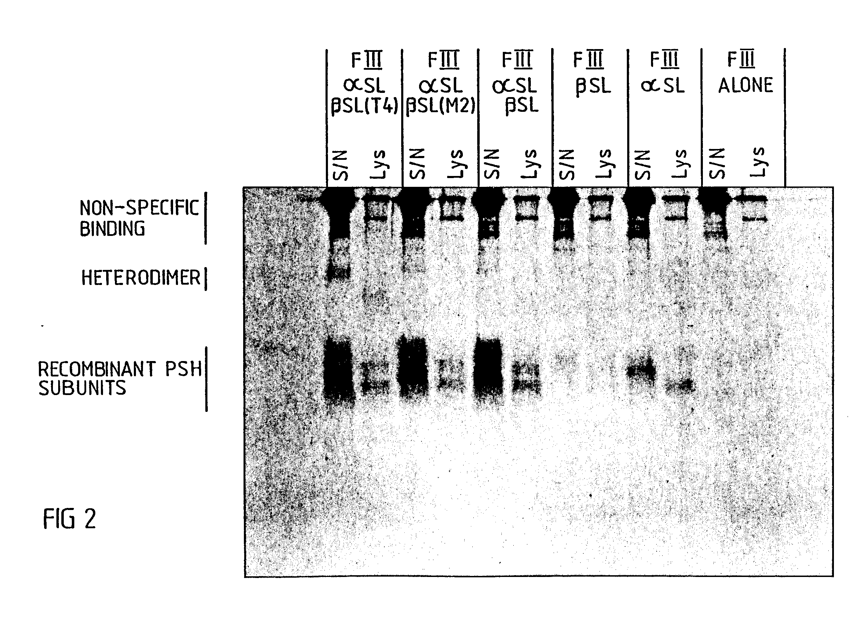

Cell lysates and supernatants for each transfection were immuno-precipitated with a mixture of anti-ovine FSH monoclonal antibodies and anti-ovine FSH polyclonal antisera and analysed by SDS-PAGE (see Figure 2).

Autoradiographs of the gel show increases in molecular weight between lysates (Lys) and supernatants (S/N) for both alpha and beta subunits as a result of glycosylation. Some non-dissociated or reassociated heterodimer is evident in the lysates and supernatant of the FIII αSL/FIIIβSL(T4) transfected cells. Subunits were dissociated prior to loading of samples by boiling in non-reducing buffer.

## CHO Cell Transfection for Constitutive Expression of Recombinant FSH

### Transfection 1

Six different vector combinations were transfected by Lipofectin mediated DNA transfection into CHO cells. These were
(i) FIII vector alone.
(ii) FIII αFL + FIII βFL + pMC1 neoPolyA
(iii) FIII αFL + FIII βSL + pMC1 neoPolyA
(iv) FIII αSL + FIII βSL + pMC1 neoPolyA
(v) FIII αFL + pMC1 neoPolyA
or
(vi) FIII βSL + pMC1 neoPolyA.

The vector pMC1 neoPolyA was included to confer drug resistance to G418 as a selectable marker for transfected cell lines. The pMC1 neoPolyA vector contains an active neomycin resistance gene which confers resistance to G418. Colonies of resistant cells were visible 10 days after transfection in all except the negative control transfection containing no pMC1 neoPolyA vector. Colonies were released from the tissue culture dishes, replated and left to grow until confluent. Half of each cell pool was frozen for storage while the remaining cells were expanded, grown to confluence and induced with 75mM ZnSO$_4$. Three days later supernatants were collected and assayed by radio receptor assay (RRA) (Maghuin-Rogister 1978). This assay is specific for the alpha/beta heterodimer, individual subunits having less than 1% reactivity. Some non-specific inhibition was seen when neat supernatants were assayed. This was most probably due to the 10% foetal calf serum supplement in the growth media. RRA results (see Table 3) show all three alpha/beta transfections resulted in production of rFSH with receptor binding activity. Individual subunit transfections were negative for recombinant FSH in this assay. The combination of both truncated cDNA subunit constructs showed the highest rate of secretion.

TABLE 3

| Radio-receptor assay results for transfected CHO cell pool supernatants for Transfection 1. | | | |
|---|---|---|---|
| Standard/Sample | | Counts Bound | Est. ng/ml rFSH in Tissue Culture Supernatant |
| 1000 ng/ml Standard<br>200 ng/ml Standard<br>40 ng/ml Standard<br>8 ng/ml Standard<br>1.6 ng/ml Standard<br>"0" Standard | | 958<br>1204<br>2061<br>3937<br>5953<br>6465 | |
| FIII αFL + FIII βFL | (Neat)<br>(1:3.5) | 4446<br>5669 | >4.2 |
| FIII αFL + FIII βSL | (Neat)<br>(1:3.5) | 4500<br>5800 | >4.2 |
| FIII αSL + FIII βSL | (Neat)<br>(1:3.5) | 3290<br>5000 | >9.0 |
| FIII αFL | (Neat)<br>(1:3.5) | 5494<br>6358 | <1.0 |
| FIII βSL | (Neat)<br>(1:3.5) | 5637<br>6442 | <1.0 |
| ( ) = Tissue Culture supernatant sample dilution factors for radioreceptor assay. | | | |

Transfection 2

Ten different vector combinations were transfected by Lipofectin mediated DNA transfection into CHO cells and expanded for RRA as for Transfection 1 above.

These were

    (i) FIII vector alone

    (ii) FIII αFL + FIII βSL + pMCI neoPolyA

    (iii) FIII αSL + FIII βFL = pMCI neoPolyA

    (iv) FIII αSL + FIII βSL + pMCI neoPoly

    (v) FIII αSL + FIII βSL(M2) + pMCI neoPolyA

    (vi) FIII αSL + FIII βSL(M12) + pMCI neoPolyA

    (vii) FIII αSL + FIII βSL(T4) + pMCI neoPolyA

    (viii) FIII αSL + pMCI neoPolyA

    (ix) FIII βSL + pMCI neoPolyA

    (x) Human αFSH construct + human βFSH + pMCI neoPolyA.

Results for each vector combination transfection pool are given in Table 4.

## TABLE 4

Radio-receptor assay results for transfected CHO cell pool supernatants for transfection 2.

| Standard/Sample | | Counts Bound | Est. ng/ml rFSH in Tissue Culture Supernatant |
|---|---|---|---|
| 1000 ng/ml Standard | | 1411 | |
| 200 ng/ml Standard | | 1590 | |
| 40 ng/ml Standard | | 1989 | |
| 8 ng/ml Standard | | 3219 | |
| 1.6 ng/ml Standard | | 5860 | |
| 0.3 ng/ml Standard | | 7959 | |
| "0" Standard | | 9107 | |
| FIII αFL + FIII βSL | (1:3) | 8619 | <1 ng/ml |
| + pMC1 neoPolyA | (1:6) | 9144 | |
| FIII αSL + FIII βFL | (1:3) | 7700 | <1 ng/ml |
| + pMC1 neoPolyA | (1:6) | 8282 | |

**Table 4 continued**

| Standard/Sample | | Counts Bound | Est. ng/ml rFSH in Tissue Culture Supernatant |
|---|---|---|---|
| FIII αSL + FIII βSL | (1:3) | 7300 | ≈2 ng/ml |
| + pMC1 neoPolyA | (1:6) | 8213 | |
| FIII αSL + FIII βSL(M2) | (1:3) | 7265 | ≈2 ng/ml |
| + pMC1 neoPolyA | (1:6) | 8175 | |
| FIII αSL + FIII βSL(M12) | (1:3) | 7855 | <2 ng/ml |
| + pMC1 neoPolyA | (1:6) | 8511 | |
| FIII αSL + FIII βSL(T4) | (1:3) | 7124 | ≈2 ng/ml |
| + pMC1 neoPolyA | (1:6) | 7858 | |
| FIII αSL + pMC1 neoPolyA | (1:3) | 8365 | <1 ng/ml |
| | (1:6) | 8452 | |
| FIII βSL + pMC1 neoPolyA | (1:3) | 8583 | <1 ng/ml |
| | (1:6) | 8831 | |
| Human αFSH construct + human βFSH construct + pMC1 neoPolyA | (1:3) | 8037 | ≈1ng/ml |
| | (1:6) | 8281 | |

( ) = Tissue Culture supernatant sample dilution factors for radioreceptor assay.

The three highest secreting transfected CHO cell pools were plated on 96 well tissue culture plates at a dilution permitting the isolation of single cells for colony isolation. After 14 days 48 wells of each transfection containing mostly single and some double colonies were assayed for recombinant FSH production. The six highest producing clones of each transfection pool were replated, allowed to grow to confluence and reassayed without Zinc induction. The two highest producing clones were replated on 96 well plates to enable the selecdtion of cloned cell lines.

Radio-receptor assay results for the 2 highest rFSH secreting clones isolated from each of 3 different vector combination transfections are given in Table 5. the supernatants assayed were for non-induced semi-confluent cell cultures.

TABLE 5

| RRA results for the 2 highest rFSH secreting clones isolated from each of 3 different vector combination transfections of Transfection 2. | | | |
|---|---|---|---|
| Standard/Sample | | Counts Bound | Est. ng/ml rFSH in Tissue Culture Supernatant |
| 1000 ng/ml Standard | | 1703 | |
| 200 ng/ml Standard | | 2060 | |
| 40 ng/ml Standard | | 2515 | |
| 8 ng/ml Standard | | 2924 | |
| 1.6 ng/ml Standard | | 3036 | |
| "0" Standard | | 2992 | |
| FIII αSL + FIII βSL + pMCI neoPolyA | Clone 1 | (1:4) 2687 | >128 ng/ml |
|  | Clone 9 | (1:4) 2435 | >640 ng/ml |
| FIII αSL + FIII βSL (M2) + pMCI neoPolyA | Clone 4 | (1:4) 2664 | >128 ng/ml |
|  | Clone 30 | (1:4) 2553 | >128 ng/ml |
| FIII αSL + FIII βSL (T4) + pMCI neoPolyA | Clone 14 | (1:4) 2148 | >640 ng/ml |
|  | Clone 30 | (1:4) 2282 | >640 ng/ml |
| ( ) = Tissue Culture supernatant sample dilution factors for radioreceptor assay. | | | |

Summary

Recombinant FSH subunit peptides have been synthesised and secreted individually and as heterodimers in vitro as demonstrated by immunoprecipitation of metabolically labelled peptides of correct molecular weight, and by inhibition of iodinated FSH receptor binding in testis homogenate radio receptor assays.

References

(1) Sairam, M.R., Seidah, N.G. and Chretien, M. (1981) Biochem. J. 197: 541-552

(2) Sanger, F., Nicklen, S. and Coulson, A.R. (1977) Proc. Natl. Acad. Sci. USA. 74: 5463-5467.

(3) Henikoff, S. (1984) Gene 28: 351-359

(4) Maurer, R.A. and Beck, A. (1986) DNA 5: 363-369

(5) Bello, P.B., et al. Nucl. Acids Res. 17(24) 10494 (1989)

(6) Mountford, P.S. et al. Nucl. Acids Res. 17(15) 6391 (1989)

(7) Sairam, M.R. Biochem. J. 197, 535-539 (1981)

(8) Sairam, M.R. et al. Biochem. J. 197, 541-552 (1981)

(9) Freidman, J.S. et al. Bio/Tech. 7, 359-362 (1989)

(10) Kozak, M. Cell 44, 283-292 (1986)

(11) Shaw, G. et al. Cell 46, 659 (1986)

(12) Gluzman, Y. Cell 23, 175 (1981)

(13) Maghuin-Rogister, G. et al. Euro. J. Biochem. 86, 121-131 (1978)

(14) Felgner, P.L. et al. PNAS 84, 7413-7417 (1987)

Finally, it is to be understood that various other modifications and/or alterations may be made without departing from the spirit of the present invention as outlined herein.

## Claims

1. A process for the preparation of a molecular clone which process includes
providing
a complementary DNA (cDNA) sequence, genomic DNA sequence or fragment thereof coding for the alpha and/or beta sub-unit of ovine follicle stimulating hormone; and
a suitable cloning vector;
ligating the DNA sequence and cloning vector to insert the DNA sequence into the cloning vector to form a molecular clone.

2. A process according to claim 1 wherein the complementary DNA sequence, genomic DNA sequence or fragments thereof includes a complete or partial copy of the alpha and/or beta subunit of ovine follicle stimulating hormone coding regions encompassing the mature hormone, extending past the 3' termination codon.

3. A process according to claim 2 wherein the cloning vector is a bacteriophage vector.

4. A process according to claim 3 wherein the bacteriophage vector is lambda gt 10.

5. A process according to claim 3 including the preliminary step of
providing a source of ovine lamb pituitary gland;
isolating polyadenylated messenger RNA (mRNA) therefrom;
treating the mRNA with reverse transcriptase in the presence of oligodT primer to synthesize first strand complementary DNA (cDNA);
enzymatically removing the mRNA with RNase H, and subsequently synthesising the second cDNA strand with DNA polymerase I; and
treating the double stranded complementary DNA (cDNA) so formed with T4 DNA polymerase to create flush (blunt) ended molecules.

6. A process according to claim 2 including the preliminary step of
providing a source of ovine DNA isolated from sheep genome; and
enzymatically cleaving the genomic DNA with a suitable enzyme.

7. A process according to claim 5 or 6 further including;
providing a source of virus particles; and
a bacterial carrier strain
packaging the DNA sequences into the virus particles "in vitro";
infecting the bacterial carrier strain with the virus particles to form a DNA library; and
selecting recombining clones containing DNA sequences coding for the α and/or β sub-unit of ovine FSH.

8. A bacteriophage clone selected from the clones 3.2 and 6.14 as hereinbefore described.

9. A plasmid expression vector including a cDNA sequence, genomic DNA sequence or fragments thereof coding for the alpha-subunit ovine FSH and capable of being replicated, transcribed and translated in a prokaryotic or eukaryotic organism.

10. A plasmid expression vector as claimed in Claim 9 selected from FIII αFL and FIII αSL as hereinbefore described.

11. A plasmid expression vector including a cDNA sequence, genomic DNA sequence or fragments thereof coding for the beta-subunit of ovine FSH and capable of being replicated, transcribed and translated in a prokaryotic or eukaryotic organism.

12. A plasmid expression vector as claimed in claim 11 selected from FIII βFL, FIII βSL, FIII βSL(M2), FIII βSL(M12) and FIII βSL(T4) as hereinbefore described.

13. A plasmid expression vector including
a first cDNA sequence, genomic DNA sequence or fragments thereof coding for the alpha-subunit of ovine FSH, and
a second cDNA sequence, genomic DNA sequence or fragments thereof coding for the beta-subunit of ovine FSH; and
capable of being replicated, transcribed and translated in a prokaryotic or eukaryotic organism.

14. A process for the preparation of a recombinant polypeptide having ovine FSH activity which process includes the steps of
providing
a first recombinant plasmid including a plasmid expression vector including a complementary DNA (cDNA) sequence coding for the alpha sub-unit ovine FSH and capable of being replicated, transcribed and translated in a prokaryotic or eukaryotic organism,
a second recombinant plasmid including a plasmid expression vector including complementary DNA (cDNA) sequence coding for the beta sub-unit ovine FSH and capable of being replicated, transcribed and

17

EP 0 404 458 A2

translated in a prokaryotic or eukaryotic organism,
a prokaryotic or eukaryotic organism;
introducing said first and second recombinant plasmids into said organism by a method selected from transformation, transfection, injection or electroporation;
culturing the resulting organism;
expressing the recombinant polypeptide encoded by said DNA sequences; and optionally
isolating said polypeptide from the culture or organism.

15. A process according to claim 14 wherein the plasmid expression vector is a vector capable of expressing a cDNA sequence, genomic DNA sequence or fragments thereof and including a promoter sequence capable of modulation by trans-acting molecules.

16. A process according to claim 15 wherein the regulatory element is a metallothionein promoter.

17. A process according to claim 15 wherein the plasmid expression vector further includes an upstream SV40 enhancer element.

18. A process according to claim 15 wherein the plasmid expression vector further includes an SV40 derived BamH1 fragment containing the small (t) intron and polyadenylation signal.

19. A process according to claim 18 wherein the plasmid expression vector is the vector FIII, as hereinbefore described.

20. A process according to claim 14 wherein the first recombinant plasmid includes a cDNA sequence including a 717 bp EcoR1 - EcoR1 fragment including the coding region for the alpha subunit of ovine follicle stimulating hormone and wherein the plasmid is FIIIαFL or a derivative or variant thereof as hereinbofore described.

21. A process according to claim 20 wherein the cDNA sequence is reduced in length and wherein the plasmid is plasmid FIIIαSL, or a derivative or variant thereof, as hereinbefore described.

22. A process according to claim 14 wherein the second recombinant plasmid includes a cDNA sequence including a 1589 bp EcoR1-EcoR1 fragment including the coding region for the beta subunit of ovine follicle stimulating hormone and wherein the plasmid is plasmid FIIIβFL or a derivative or variant thereof as hereinbefore described.

23. A process according to claim 22 wherein the cDNA sequence is reduced in length and wherein the second recombinant plasmid is plasmid FIIIβSL, or a derivative or variant thereof as hereinbefore described.

24. A process according to claim 23 wherein the cDNA sequence is further modified to increase the translatability of the corresponding messenger RNA (mRNA).

25. A process according to claim 23 wherein the cDNA is further modified including incorporating into the signal peptide region of the cDNA sequence an optimized translation initiation DNA sequence.

26. A process according to claim 34 wherein the second recombinant plasmid is plasmid FIIIβSL(M2) or a derivative or variant thereof as hereinbefore described.

27. A process according to claim 34 wherein the second recombinant plasmid is plasmid FIIIβSL(M12) or a derivative or variant thereof as hereinbefore described.

28. A process according to claim 23 wherein the cDNA sequence is modified by replacing the 5′ non-coding region and signal peptide coding region of teh beta subunit of ovine follicle stimulating hormone with the equivalent region of ovine growth hormone cDNA.

29. A process according to claim 28 wherein the second recombinant plasmid is plasmid FIIIβSL(T4) as hereinbefore described.

30. A process according to claim 14 wherein the unicellular organism is a strain of E. coli, E. coli NM 514.

31. A process according to claim 14 wherein the eukaryotic organism is a transgenic animal.

32. A plasmid including
a plasmid expression vector;
a first DNA sequence encoding a desired protein; and
a second non-related DNA sequence including a translation initiation and optionally transcription initiation region;
a 5′ non-coding region; and
a signal peptide coding region; the second DNA sequence being positioned upstream of said first DNA sequence.

33. A plasmid according to claim 32 wherein the second DNA sequence is derived from a DNA sequence encoding a signal peptide.

34. A plasmid according to claim 33 wherein the peptide is a growth hormone signal peptide, preferably an ovine growth hormone signal peptide.

35. A plasmid according to claim 32 wherein the first DNA sequence is a sequence encoding for a

18

follicle stimulating hormone; or a fragment thereof, preferably ovine follicle stimulating hormone.

36. A veterinary composition including recombinant polypeptide having ovine growth hormone activity as hereinbefore described and the veterinarily acceptable salts thereof together with one or more carriers acceptable for veterinary use.

37. A method of inducing or increasing ovulation in female animals which method includes administering to the animal an effective amount of a ovine synthetic FSH, or derivative thereof, as hereinbefore described.

38. A method of increasng the number of ova produced during ovulation in female animals which method includes administering to the animal a synthetic ovine follicle stimulating hormone (FSH), as hereinbefore described wherein the animal is a bovine animal the hormone is administered in a total amount of from approximately 20 to 60 U in at least three substantially equal doses at at least three regular intervals during a period of 1 day, dependant upon the size of the animal; and

when the animal is an ovine or caprine animal the hormone is administered in an amount of from approximately 20 to 40 U in a single dose.

39. A method according to claim 38 wherein the animal is a sheep or goat and synthetic ovine FSH is administered in amount of approximately 20 to 30 U in a single dose.

40. A method according to claim 38 wherein the animal is bovine and ovine FSH is administered in three substantially equal amounts totalling approximately 0.06 to 0.24 U/kg live weight in a single day.

41. A method according to claim 40 wherein the total dose rate is approximately 0.09 to 0.15 U/kg live weight in a single day.

42. A method according to claim 38 further including the preliminary step of administering to the animal approximately 250 ug to approximately 100 mg of a synchronising agent approximately 8 to 15 days prior to initiation of synthetic ovine FSH treatment.

43. A method according to claim 38 further including the step of administering to the animal an amount of approximately 250 ug to approximately 1000 ug of a prostaglandin or prostaglandin approximately 1 to 3 days after the initiation of synthetic ovine FSH treatment.

44. A method according to claim 43 further including the step of administering to the animal approximately 100 I.U. to 1000 I.U. of gonadotrophin of pregnant mare serum gonadotrophin and is administered to the animal during the first day of ruminant follicle-stimulating hormone treatment.

45. A veterinary composition including an effective amount of synthetic ovine follicle-stimulating hormone, (as hereinbefore described) and an effective amount of a veterinarily acceptable carrier or excipient therefore, for use in the method according to claim 37.

46. A veterinary composition according to claim 45 wherein the veterinary composition is in an injectable form and includes an effective amount of synthetic ovine follicle stimulating hormone in a lyophilised form and an effective amount of an aqueous solvent therefor.

47. A kit of parts including a supply of a synthetic ovine follicle-stimulating hormone (as hereinbefore defined) in a lyophilised form in a suitable container; and a supply of an aqueous solvent therefor in a suitable container; and a supply of an aqueous solvent therefore in a suitable container for use in a method according to claim 37; and

further including a supply of a synchronising agent in a suitable container; a supply of luteinising agent in a suitable container; and

a supply of a gonadotrophin or derivative thereof in a suitable container.

```
                                                          -19                                                                    1
                                                           M  K  S  V  Q  F  C  F  L  F  C  C  W  R  A  I  C  C  R  S  C  E
ACAAGGCAGCTGTCTACGGAAAAGTCTCAGCATCCACAGTTACCAAGTGCCCAGGATGAAGTCCGTCCAGTTCTGCTTCCTTTTCTGTTGCTGGAGAGCAATCTGCTGCAGAAGCTGCGA    120
    L  T  N  I  T  I  T  V  E  K  E  E  C  S  F  C  I  S  I  N  T  T  W  C  A  G  Y  C  Y  T  R  D  L  V  Y  K  D  P  A  R
GCTGACCAACATCACCATCACGGTGGAGAAAGAGGAATGTAGCTTCTGCATAAGCATCAACACCACGTGGTGTGCAGGCTATTGCTACACCCGGGACTTGGTGTACAAGGACCCAGCGAG    240
       P  N  I  Q  K  A  I  C  T  F  K  E  L  V  Y  E  T  V  K  V  P  G  C  A  H  H  A  D  S  L  Y  T  Y  P  V  A  T  E  C  H  C
GCCCAACATCCAGAAAGCATGTACCTTCAAGGAGCTGGTGTACGAGACGGTGAAAGTGCCTGGCTGTGCCCACCATGCAGACTCCCTGTACACGTACCCAGTAGCCACTGAATGTCACTG    360
    G  K  C  D  R  I  D  S  T  D  C  T  V  R  G  L  G  P  S  Y  C  S  F  S  D  I  R  E  *
CGGCAAGTGTGACCGCGACAGCACTGACTGCACCGTGCGAGGCCTGGGGCCCAGCTACTGCTCCTTCAGTGACATCAGAGAATAAAGAGCAGCAGCTGCTTTGAGCTGCCTACCCTTAAA    480
GGACCAAAACATCCAAGATGTCTGTGTGTACATGCGCCTAGGCTGCAGACCACCACAGAAGACCCTACTGATCTCTGCTCTCCTGAGAAGGGGAGCTCCAGGAATGGAGAGGGCTGGGGC    600
CTGGCACCACTCAGACCTGTATTCTACATCTGGTTCCATGAGTCTTACTCACTCTCACTTAACTTACAGACACGAGGGTGCTTTTCCATTTAATAATCTTAGAAATCCTCTCAGGCAATC    720
CCTTCCTCTTAAAGCTAGGGATACGGTCCCAGGGGAAGGAAATGAGCTAAAAGTAGGTGAAAGTCCTAAATGCAGCATTCTCCTGCCAGACTCAAGAGCCCTCAAGAGAAGAGCCAACCT    840
CTTCTTCATGATTGTAGCCTCAATGCCTAACACTCCACATAGAATTTAGTAAGAAACTTAATAATGGCTTCCTTAAAGAAAGTAAGATCAGAAAGTTAGGGATAGGAGAACACTGAGAGA    960
GGATGTTAGAGGCTATCTGATAGGGCTGCGCTCCACGGAAGGGGGGCATCAGCTTCAGTTCTGTCTCGGCTCTCCTCCTCTGGCACATGGAGGATGGAGACAGGATAAAACTCTCTGAAT   1080
CATTCAGGCAGGTGGCAGTTACCAAGCTCAGGATTTCCAAGCTATTACTGCCGAGTCCTTTAGTTAAGGGCAAAAGCAAGAAATATTAATACGGGTTTGTGGAAATTAGCTGCATCTATT   1200
CACTTTTCACTTTCATGCATTGGAGAAGGAAATGGCAACCCACTCCAGTGTTCTTGCCTGGAGAATCCCAGGGACGGGGGAGCCTGGTGGGCTGCCATCTATGAGGTTGCAGAGGGTCGG   1320
ACACGACTGAAGTGACTTAGCAGCAGCATTCCATCATTTAAATAAATGGAACAAATGCTGTCAGGCTCCTGCAAAACCCTTTCGGGAAGTACATAGATCTATATCACCCTGCCCTTGAAT   1440
GTAGGCTCTACTCCTTTGGAAAGAGGTGGATTTGAGAGCAGGTTTGAAAGCAATTTTGACAACTTAATAAGTACATTTATCTTATCTTCAAATACATTTATGTAAAGAAAATGCTCTTTT   1560
AGAAAGAATTAGCCATAACAAAAAAAAAA   1589
```

# FIG 1A

EP 0 404 458 A2

EP 0 404 458 A2

```
                                                              -24
                                                     M  D  Y  Y  R  K  Y  A  A
TTTTTTTCTTCTTCAGATCCACAATCAACTCTCCTGACTACATTCTGCAAAAAATTCAGAGAACGAAGAGCCATGGATTACTACAGAAAATATGCAGCTG      155


                                          -1  +1
V  I  L  A  I  L  S  L  F  L  Q  I  L  H  S  F  P  D  G  E  F  T  M  Q  G  C  P  E  C  K  L  K  E  N
TCATTCTGGCCATATTGTCTCTGTTTCTGCAAATTCTCCATTCCTTTCCTGATGGAGAGTTTACAATGCAGGGTTGTCCTGAATGCAAGCTAAAAGAAA      255


   K  Y  F  S  K  P  D  A  P  I  Y  Q  C  M  G  C  C  F  S  R  A  Y  P  T  P  A  R  S  K  K  T  M  L
CAAATACTTCTCCAAGCCAGATGCTCCAATTTATCAGTGCATGGGGTGCTGCTTCTCCAGGGCATACCCCACTCCAGCGAGGTCTAAGAAGACAATGTTG      355


V  P  K  N  I  T  S  E  A  T  C  C  V  A  K  A  F  T  K  A  T  V  M  G  N  V  R  V  E  N  H  T  E
GTTCCCAAGAACATCACCTCGGAAGCCACATGTTGTGTGGCCAAAGCATTTACCAAGGCCACAGTGATGGGAAATGTCAGAGTGGAGAACCACACCGAGT      455


            96
C  H  C  S  T  C  Y  Y  H  K  S
GCCACTGCAGTACTTGTTATTATCACAAATCCTAAATATTTTGCAGTGGGCCATGTTGATGATGATGGTTGATTTGCTCAAAAGGAAAATTAATTTGTCC      555


AGTGTCTATGCCTTTGTGAGATAAAACCCTCCTTTTCCTTGCCGTACATTTTTAACCTGCTTTGAGAATATACTGCAGCTTTATTGCTTTTCTCCTTATC      655


CTACAATATAATCAGCAGTCTTGATCTTTTCATTTGGAATGAAAATATGGCATTTAGCATGACCATAAAAAGCTGGTTCCACTGGAAATAAAGTATTTTA      755

AATCATCAAAAAAAAA  771
```

<div align="center">FIG 1B</div>

FIG 2